# EUROPEAN PATENT APPLICATION

(11) **EP 4 593 027 A1**
(43) Date of publication of application: **30.07.2025**
(21) Application number: 23894844.2
(22) Date of filing: 27.10.2023
(51) Int. Cl.: G16H 30/40, G16H 30/20, G06V 10/82, G06V 10/75, G06T 3/40, A61B 5/055, A61B 6/03, G06N 3/08

(54) **METHOD FOR CONVERTING MEDICAL IMAGES BY MEANS OF ARTIFICIAL INTELLIGENCE BY USING IMAGE QUALITY CONVERSION AND DEVICE THEREFOR**

(30) Priority: 23.11.2022 KR 20220158715
(71) Applicant: Polestar Healthcare Co., Ltd., Seoul 06627 (KR)
(72) Inventor: YOON, Yeo Dong, Seoul 06629 (KR); LEE, Eun Chong, Yongin-si Gyeonggi-do 16812 (KR); NA, Eun Su, Bucheon-si Gyeonggi-do 14591 (KR)
(74) Representative: RGTH
(86) International application number: PCT/KR2023/016875
(87) International publication number: WO 2024/111915

(57) **Abstract**

An aspect of the present invention relates to a method for converting medical images and, more specifically, to an image conversion method for converting medical images by using a generative adversarial network (GAN). An embodiment of the present invention provides a new model learning method wherein a learning model is trained to intentionally generate a blurred target image obtained by blurring a target image and to compare and distinguish the blurred target image with the target image such that an output image having an improved contrast ratio can be obtained.

## Description

### TECHNICAL FIELD

An aspect of the disclosure relates to a method and device for converting a medical image by artificial intelligence (Al) using image quality conversion and, more specifically, to a method and device for converting a medical image using a generative adversarial network (GAN) to improve the clarity of an output image by reducing or improving the image quality.

### BACKGROUND ART

The information disclosed in this section is only provided for an understanding of background information of embodiments of the disclosure and should not be taken as a description of the prior art.

In recent years, the conversion of medical images has emerged as an important issue in the field of radiology for medical professionals to diagnose and treat patients.

For the diagnosis and treatment of emergency room patients with brain hemorrhage, tumor patients, and the like, medical professionals may use non-enhanced (or non-contrast enhanced) CT images in which no contrast enhancers are used, enhanced (or contrast enhanced) CT images in which contrast enhancers are used, and the like.

In the case of enhanced CT images, for example, a contrast agent for enhancing tissue contrast is useful for detecting blood vessels, organs, cancerous tumors, and the like.

However, contrast agents may often cause side effects and even death due to cardiac arrest, shock death, or the like.

Because the side effects of contrast agents may not be predicted in advance, image conversion of converting non-enhanced CT or MR images to enhanced CT or MR images may help prevent these side effects and reduce the medical costs of contrast agents.

In addition, for individuals such as pregnant women who have MR images but no CT images due to unavailability of X-ray radiation or individuals who have CT images but are not MR imaged for reasons such as lack of time, cost, or implants, image conversion between imaging modalities may also help medical professionals to identify tissue boundaries or locate tumors without the use of contrast agents.

Furthermore, conversion between MR images, such as from a T1 image to a T2 image or from a T1 image to a diffusion image, may help medical professionals better understand lesions.

In addition, conventional Al learning models is trained to reduce the difference between an output image produced as a result and a target image by comparing the output image and the target image. In order for medical professionals to analyze the medical image, the output image produced by the learning model must be precise enough to match the target image accurately, but the actual learning model tends to determine that the output image matches the target image even in a case in which the output image is blurred. Therefore, the problem is low quality of the output result.

In particular, in the case of a medical image, the resolution of an output image must be high enough because the medical image serves as a basis for accurate judgment by medical professionals. Therefore, it is urgent to apply new training methods to Al learning models to produce clear output images.

The information disclosed in the Background section is technical information that the inventors possessed for, or acquired during, derivation of embodiments of the disclosure and should not be taken as known technology disclosed to the public before the filing of the embodiments of the disclosure.

### DISCLOSURE

### Technical Problem

Accordingly, an aspect of the disclosure has been made to solve the above-described problems, and an objective of the disclosure is to provide a new model training method which may obtain an output image with improved clarity by training a learning model to additionally construct a blurred target image from a target image and to compare and discriminate the blurred target image with and from the target image.

Another objective of the disclosure is to provide a method for converting a medical image which enables medical professionals to perform medical examination, diagnosis, and treatment with accurate information and patients to receive appropriate medical services.

The objectives of the disclosure are not limited to the foregoing description, and other objectives not explicitly disclosed herein will be clearly understood by a person having ordinary knowledge in the art to which the disclosure pertains from the description provided hereinafter.

### Technical Solution

In order to achieve at least one of the above objectives, an aspect of the present disclosure provides a model training method for medical image conversion which converts a medical image using a generative adversarial network (GAN), the model training method including:
a first operation of receiving a first original image selected from a paired data set including the first original image and a second original image;
a second operation of constructing an output image based on the first original image, the output image matching the first original image and belonging to a different domain from the first original image;
a third operation of degrading quality of the second original image to construct a degraded image; and
a fourth operation of performing training based on the degraded image to improve accuracy of a result of medical image conversion.

In some embodiments, in the third operation, the degraded image may be constructed by blurring the second original image.

In some embodiments, the blurring may include removing high frequency components from the second original image.

In some embodiments, the fourth operation may include: a first discrimination operation of discriminating the output image from the second original image; and a second discrimination operation of discriminating the degraded image from the second original image.

In some embodiments, in the first discrimination operation, the training may be performed to determine that the output image is different from the second original image, and in the second discrimination operation, the training may be performed to determine that the degraded image is different from the second original image.

In order to achieve at least one of the above objectives, another aspect of the present disclosure provides a device for converting a medical image using a generative adversarial network (GAN), the device including:
an image quality degrader configured to degrade image quality of a second original image selected from a paired data set including a first original image and the second original image to construct a degraded image; and
a learning model configured to receive the first original image,
construct an output image based on the first original image, the output image matching the first original image and belonging to a different domain from the first original image,
receive the degraded image from the image quality degrader, and be trained based on the degraded image to improve accuracy of a result of medical image conversion.

In some embodiments, the image quality degrader may construct the degraded image by blurring the second original image.

In some embodiments, the blurring may include removing high frequency components from the second original image.

In some embodiments, the learning model may include a first discriminator configured to be trained to discriminate the output image from the second original image and a second discriminator configured to be trained to discriminate the degraded image from the second original image.

In some embodiments, the first discriminator may be trained to determine that the output image is different from the second original image, and the second discriminator is trained, like the first discriminator, to determine that the degraded image is different from the second original image.

In order to achieve at least one of the above objectives, another aspect of the present disclosure provides an artificial intelligence (AI) learning model which includes a constructor and a discriminator and is trained by machine learning, the Al learning model including:
a constructor configured to receive a first original image and be trained to construct an output image based on the first original image using a generative adversarial network (GAN);
a first discriminator configured to be trained to discriminate the output image constructed by the constructor from a second original image paired with the first original image; and
a second discriminator configured to be trained to discriminate the degraded image degraded from the second original image from the second original image.

In some embodiments, the degraded image may be constructed by blurring the second original image.

In some embodiments, the blurring may include removing high frequency components from the second original image.

In some embodiments, the first discriminator may be trained to determine that the output image is different from the second original image, and the second discriminator is trained, like the first discriminator, to determine that the degraded image is different from the second original image.

In order to achieve at least one of the above objectives, another aspect of the present disclosure provides a model training method for medical image conversion which converts a medical image using a generative adversarial network (GAN), the model training method including:
a first operation of receiving a first original image selected from a paired data set including the first original image and a second original image;
a second operation of constructing an output image based on the first original image, the output image matching the first original image and belonging to a different domain from the first original image;
a third operation of improving quality of the second original image to construct an improved image; and
a fourth operation of performing training based on the improved image to improve accuracy of a result of medical image conversion.

### Advantageous Effects

According to an embodiment of the disclosure, a new model training method may obtain an output image with improved clarity by training a learning model to additionally construct a blurred target image from a target image and to compare and discriminate the blurred target image with and from the target image.

Furthermore, a method for converting a medical image may enable medical professionals to perform medical examination, diagnosis, and treatment with accurate information and patients to receive appropriate medical services.

In addition, the disclosure has a variety of effects with excellent versatility according to embodiments, and such effects may be clearly understood from the following description of embodiments.

### DESCRIPTION OF DRAWINGS

The following drawings accompanying the specification illustrate embodiments of the disclosure and, together with the foregoing disclosure, serve to provide further understanding of the technical spirit of the disclosure, and thus, the disclosure should not be construed as being limited to the drawings, wherein:
FIG. 1 illustrates a method of converting a medical image according to an embodiment of the disclosure.
FIG. 2 illustrates an Al learning model according to an embodiment of the disclosure.
FIG. 3 illustrates an embodiment of calculating the match rate between an output image and a target image.
FIG. 4 illustrates a model training method according to another embodiment of the disclosure.
FIG. 5 illustrates a device for converting a medical image according to an embodiment of the disclosure.
FIG. 6 illustrates an Al learning model according to an embodiment of the disclosure.

### BEST MODE

Advantages and features of the disclosure, as well as methods of realizing the same, will be more clearly understood from the following detailed description of embodiments when taken in conjunction with the accompanying drawings. However, the disclosure is not limited to specific embodiments to be described hereinafter but should be understood as including a variety of modifications, equivalents, and alternatives within the spirit and scope of the disclosure. Rather, these embodiments are provided so that the description of the disclosure will be complete and will fully convey the scope of the disclosure to a person having ordinary skill in the art in the technical field to which the disclosure pertains. In the following description of the disclosure, a detailed description of related known technology will be omitted when the description may render the subject matter of the disclosure unclear.

The terminology used herein is for the purpose of describing particular embodiments only and is not intended to be limiting. As used herein, the singular forms are intended to include the plural forms as well, unless the context clearly indicates otherwise.

Terms, such as "comprise/include" or "have," or the like, as used herein, indicate that a feature, a number, a step, an operation, a component, a part or a combination thereof described in the disclosure is present, but do not preclude the possibility of the presence or addition of one or more other features, numbers, steps, operations, components, parts or combinations thereof in advance. Although terms, such as "first", "second", or the like, may be used to describe various components, these components should not be conceived of as being limited by these terms. These terms are only used to distinguish a component from another component.

Hereinafter, embodiments according to the disclosure will be described in detail with reference to the accompanying drawings, in which identical or similar components are given the same reference numerals, and repeated descriptions thereof will be omitted.

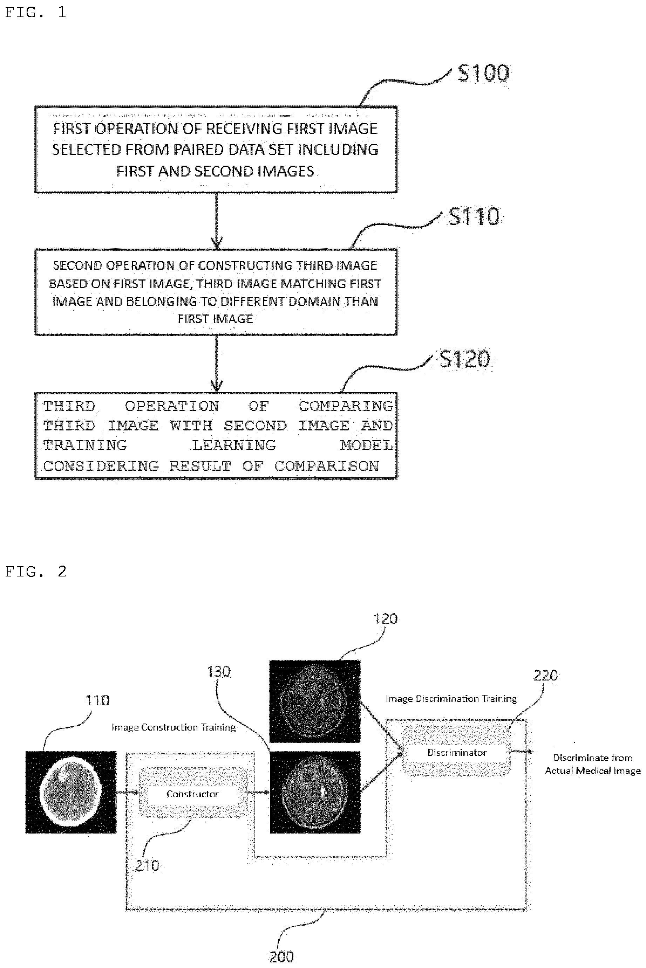

FIG. 1 illustrates a method of converting a medical image according to an embodiment of the disclosure, and FIG. 2 illustrates an artificial intelligence (AI) learning model according to an embodiment of the disclosure.

According to an embodiment of the disclosure, a method of converting a medical image using a generative adversarial network (GAN) may be provided.

The method of converting a medical image according to this embodiment may include: a first operation S100 of receiving a first image 110 selected from a paired data set including the first image 110 and a second image 120; a second operation S110 of constructing a third image 130 based on the first image 110, the third image 130 matching the first image 110 and belonging to a different domain from the first image 110;
a third operation S120 of comparing the third image 130 with the second image 120 and training the learning model considering a result of the comparison; and a fourth operation S130 of comparing the third image 130 with the first image 110 and training the learning model considering a result of the comparison.

The first image 110, the second image 120, and the third image 130 may be medical images. The medical images may be magnetic resonance images, such as 2D MR, 3D MR, 2D streaming MR, 4D MR, 4D volumetric MR, and 4D cine MR images; functional MR images, such as fMR, DCE-MR, and diffusion MR images; computed tomography (CT) images, such as 2D CT, cone beam CT, 3D CT, and 4D CT images; ultrasound images, such as 2D ultrasound, 3D ultrasound, and 4D ultrasound images; positron emission tomography (PET) images; X-ray images; fluoroscopic images; radiotherapy portal images; single-photon emission computed tomography (SPECT) images; computer generated synthetic images, such as pseudo-CT images; and the like.

Furthermore, the medical images may include medical image data, such as a training image, a ground truth image, a contoured image, a dose image, and the like.

In some embodiments, the medical image may include an image acquired using an image acquisition device, a computer generated synthetic image, and the like. The image acquisition device may include, for example, an MR imaging device, a CT imaging device, a PET imaging device, an ultrasound imaging device, a fluoroscopic device, a SPECT imaging device, an integrated linear accelerator, an MR imaging device, and the like. Furthermore, the image acquisition device is not limited to the above-described examples and may include a variety of medical imaging devices within the scope of the technical idea for acquiring medical images of a patient.

A paired data set may include a set of training data including images of an object such as a particular part of a patient.

For example, a CT imaging device and an MR imaging device may be used to acquire a CT image and an MR image, respectively, of a particular part of a patient, such as the head, in which case the CT image and the MR image may form a paired data set.

In a case in which a non-enhanced (or non-contrast enhanced) CT image and an MR image are respectively acquired for a particular part of a patient, the non-enhanced CT image and the MR image may form a paired data set. In a case in which an enhanced (or contrast enhanced) CT image and an MR image are respectively acquired, the enhanced CT image and the MR image may form a paired data set.

In a case in which T1 and T2 images are acquired from a particular part of a patient, the T1 and T2 images may form a paired data set.

In this context, the particular part of a patient may mean the particular part of a single patient. In a case in which imaged parts are the same, the imaged parts may refer to particular parts of different patients.

For example, CT and MR images acquired from a particular part of a single patient may form a paired data set, and CT and MR images acquired from the same parts of different patients may also form a paired data set.

Different domains of two images mean that the domains are different, for example, in a case in which respective patterns contained in two matching images have different contrasts or two images have different intensity distributions or different patterns.

Furthermore, images acquired using different imaging devices belong to different domains. For example, because CT and MR images are acquired using a CT imaging device and an MR imaging device, respectively, both images belong to different domains. Similarly, there are domain differences between ultrasound and CT images, between ultrasound and MR images, and between MR and PET images.

Furthermore, non-enhanced and enhanced images have different intensity distributions for the same part, and thus belong to different domains.

For MR images, T1 and T2 images are the same MR images, but have different intensity distributions and therefore belong to different domains. That is, in a case in which two images realized by reconstruction based on signals provided from an object have a difference in signals, sequences, or image realization processes on which the image realization is based, the two images having the difference belong to different domains. Furthermore, the medical image examples described above belong to different domains.

To illustrate the method of converting a medical image according to an embodiment of the disclosure, a case in which the image acquisition device has obtained a paired data set by acquiring a first image 110 and a second image 120 from the same part of a single patient will be described as an example. A case in which the first image 110 is an original CT image, the second image 120 is an original MR image, and the third image 130 is a synthetic MR image according to embodiments will be described.

The first operation S100 may include receiving, by the learning model, the first image 110 selected from the paired data set including the first image 110 and the second image 120. The first image 110 may include an input image processed from an original CT image with training data.

In the paired data set including the first image 110 and the second image 120, which one of the first image 110 and the second image 120 is to be selected as the input image may be determined by the manufacturer of medical device software which performs the method of converting a medical image according to this embodiment. In this case, which image is to be selected may be selected automatically by a selection algorithm, or may be selected manually by an operator.

The manufacturer may acquire the original CT image from the CT imaging device and the original MR image from the MR imaging device to form a paired data set, and may select the original CT image as the input image from the paired data set by an algorithm or manually.

The second operation S100 may include constructing the third image 130 based on the first image 110, in which the learning model having received the first image 110 constructs the third image 130 which matches the first image 110 and belongs to a different domain from the first image 110.

The learning model receives the original CT image as an input image and constructs a synthetic MR image based on the original CT image.

The process of the learning model constructing the third image 130 from the first image 110 which belongs to a different domain from the first image 110 may be performed by the following two processes according to embodiments. However, the process of the learning model constructing the third image 130 is not limited to the following two processes.

The first process may include one or more layers which receive the first image 110 obtained from the image acquisition device and perform convolutional operations to extract high-dimensional features having a smaller size than the first image 110, in which the layers may be represented by an artificial neural network configured to extract features. Such a feature extraction artificial neural network may receive two- or three-dimensional real image data obtained from the image acquisition device and perform convolutional operations to extract image features from the original images.

Describing this process by way of example, a convolutional layer which extracts the features of an image through a filter and a pooling layer which enhances the features and reduces the size of the image may be provided to extract the features of an image by repeating the convolution and the pooling.

In some embodiments, a convolutional layer including a plurality of layers may extract features of an input image given as an input from a first convolutional layer and produce a feature map as a result, a pooling layer may receive this feature map and produce a result having enhanced features and a reduced image size, and the output of the pooling layer may be input again into a second convolutional layer.

The second process includes one or more layers performing a deconvolution operation for the purpose of generating image data of different modalities from the output of the first process described above, in which these layers may be represented by an artificial neural network for generating images of different modalities. The artificial neural network which generates images of different modalities may perform a deconvolution operation on the result of the convolution operation of extracting the features by the artificial neural network in the first process to generate two- or three-dimensional image data of different modalities.

The third operation S120 may include operations of comparing the third image 130 with the second image 120 and training the learning model considering the comparison result. In a case in which the original CT image in the paired data set which includes the original CT image and the original MR image is determined to be the input image, the original MR image may be determined to be the target image. The second image 120 may correspond to ground truth. After constructing the third image 130, the learning model may be trained to reduce the difference between the constructed third image 130 and the second image 120 by comparing the constructed third image 130 with the second image 120. That is, the learning model may be trained to compare the synthetic MR image with the original MR image and continuously review whether the synthetic MR image is constructed to be similar to the original MR image, so that the synthetic MR image and the original MR image are the same. In some embodiments, an operation of calculating a loss value between the third image 130 and the second image 120 by applying a loss function to the algorithm may be included, and the parameters of the learning model may be updated based on the loss value.

In some embodiments, in the operation of calculating a loss value, the calculation of the loss value to update the learning model may be performed at each iteration. That is, the learning model may calculate the loss value between the synthetic MR image and the original MR image at each iteration during the training.

In a case in which the iteration includes a first iteration section and a second iteration section which are sequential, in some embodiments, the learning model may recognize overfitting and terminate the training in a case in which the absolute value of the change between the first loss value calculated in the first iteration section and the second loss value calculated in the second iteration section is less than a reference value.

In a case in which the learning model is sufficiently trained for the medical image conversion, the training may no longer be performed and be completed. The model having completed the training is now able to construct medical images as third images 130 with respect to a plurality of first images 110 having different data distributions, the third images 130 belonging to different domains from the first images 110.

A learning model 200 according to an embodiment of the disclosure may use a GAN model, and may include a constructor and a discriminator. The constructor may perform image construction training, and the discriminator may perform image discrimination training.

Referring to FIG. 2, in the learning model 200 using the GAN model, in a case in which the original CT image 110 is input as an input image to a constructor 210, the constructor 210 may be trained to construct a synthetic MR image 130 by the convolutional operation described above, and a discriminator 220 may be trained to discriminate the synthetic MR image 130 from an original MR image 120, i.e., a real medical image.

In some embodiments, the learning model 200 according to this embodiment may use a cycle GAN model (not shown). In this case, the learning model 200 may include the first constructor 210, the first discriminator 220, a second constructor, and a second discriminator. In a case in which the original CT image 110 is input to the first constructor 210 as an input image, the first constructor 210 may be trained to construct the synthetic MR image 130 through the convolutional operation described above, and the first discriminator 220 may be trained to discriminate a synthetic MR image from the original MR image 120, i.e., the real medical image. In a case in which the synthetic MR image 130 constructed by the first constructor 210 is then input to the second constructor, the second constructor may be trained to construct a synthetic CT image based on the synthetic MR image 130, and the second discriminator may be trained to discriminate a synthetic CT image from an original CT image.

Here, the original CT image 110 may correspond to the first image 110 in the method of converting a medical image described above, the synthetic MR image 130 may correspond to the third image 130, and the original MR image 120 may correspond to the second image 120.

In some embodiments, the third operation S120 may include calculating a match rate between the third image 130 and the second image 120 and feeding the calculated value back to the learning model.

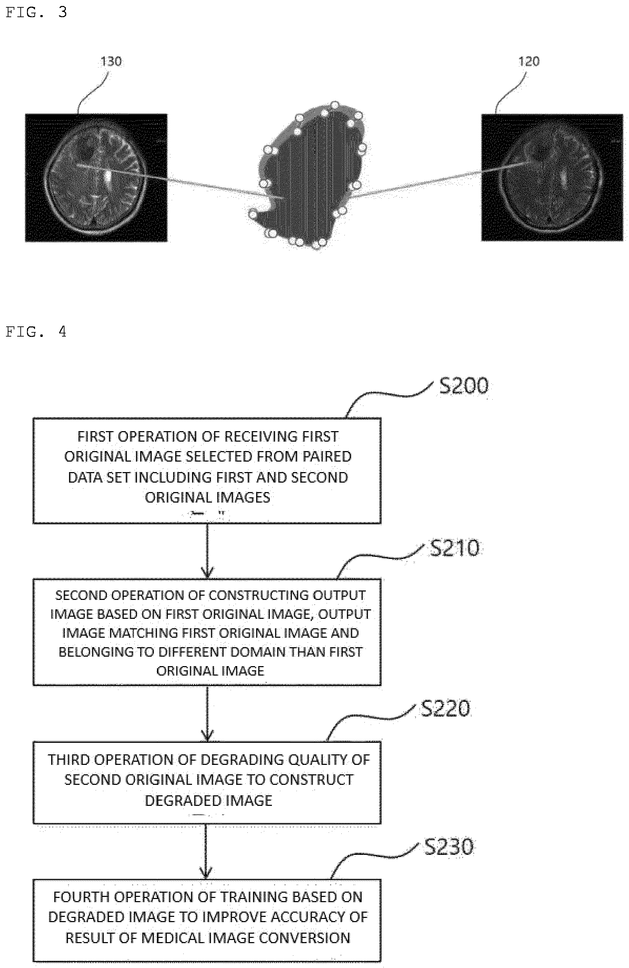

FIG. 3 illustrates an embodiment of calculating the match rate between an output image and a target image.

In some embodiments, in a case in which a lesion region is marked on each of the same parts of a synthetic MR image and an original MR image, the calculation of the match rate may be performed by comparing the sizes of the lesion regions. For example, the match rate may be calculated by marking the location of a tumor or other lesion in the synthetic MR image and the original MR image, respectively, followed by conversion to a binary image.

In this case, the match rate may be calculated using an evaluation metric, such as the Hausdorff distance and the Dice similarity coefficient, or calculated quantitatively based on the difference between the centers of mass of the two lesion locations. Furthermore, in a case in which the target image and the output image are CT images, the match rate of the two images may be determined by comparing the radiation dose calculations for the lesions. In some embodiments, the determination may be performed by representing the difference between the synthetic MR image and the original MR image using numerical values such as MAE, RMSE, SSIM, or PSNR (hereinafter referred to as a performance metric).

Referring to FIG. 3, the process of calculating the match rate by aligning the lesion regions of the synthetic MR image 130, i.e., the output image, and the original MR image 120, i.e., the target image, and comparing the contours of the respective lesions using the Hausdorff distance measure is illustrated.

FIG. 4 illustrates a model training method according to another embodiment of the disclosure.

Provided is a model training method for medical image conversion according to another embodiment of the disclosure which converts a medical image using a generative adversarial network (GAN). The model training method may include:
a first operation of receiving a first original image selected from a paired data set including the first original image and a second original image;
a second operation of constructing an output image based on the first original image, the output image matching the first original image and belonging to a different domain from the first original image;
a third operation of degrading the quality of the second original image to construct a degraded image; and
a fourth operation of performing training based on the degraded image to improve the accuracy of a result of medical image conversion.

In some embodiments, the degraded image produced in the third operation may be constructed by blurring the second original image.

In some embodiments, the blurring may include removing high frequency components from the second original image or processing through an appropriately sized image filter.

In some embodiments, the fourth operation may include: a first discrimination operation of discriminating the output image from the second original image and a second discrimination operation of discriminating the degraded image from the second original image.

In some embodiments, in the first discrimination operation, the training may be performed to determine that the output image is different from the second original image. In some embodiments, in the second discrimination operation, the training may be performed to determine that the degraded image is different from the second original image, as in the first discrimination operation.

In the third operation of the disclosure, a degraded image is constructed by intentionally degrading the second original image, i.e., the target image. The degraded image is input to the discriminator 220. The discriminator 220 is trained to compare the degraded image with the second original image and determine that the degraded image is false.

In this embodiment, both the "output image" constructed by the constructor 210 and the "degraded blurry target image" are determined to be false. The discriminator 220 is trained to determine that only the "original target image" is true.

For example, the training is performed to determine that both the blurred image and the constructed output image are false. As a result, in a case in which the "blurred image" is continuously determined to be false, the model may be trained to determine that only a "clear image" other than the "blurred blurry image" is true, such that a constructed output image is subsequently determined to be true.

The conventional cycle-GAN learning method, which does not receive the blurred target image as an input, determines only the original target image to be true and the constructed output image to be false. Thus, the learning model 200 tends to "classify" a constructed output image as the original as long as the output image is constructed similar to the original target image even though the constructed output image has somewhat low image quality.

According to the disclosure, an additional determination criterion that a blurry image with somewhat poor image quality is false is presented to the learning model 200, and one of the gists of the disclosure is to additionally determine that the blurry image is false.

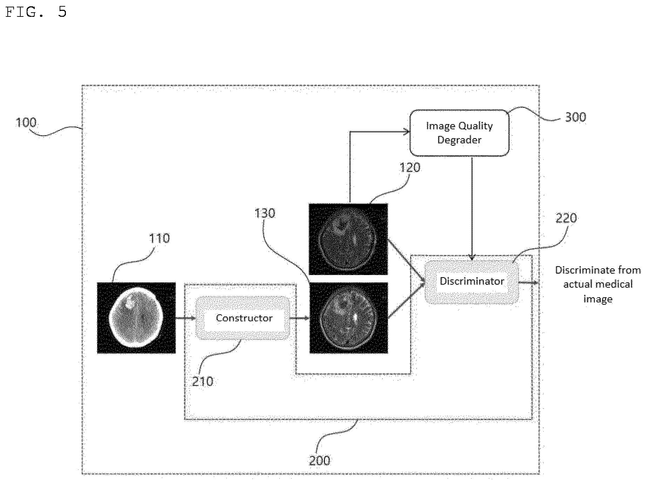

FIG. 5 illustrates a device for converting a medical image according to an embodiment of the disclosure.

Provided is a device 100 for converting a medical image according to an embodiment of the disclosure which converts a medical image using a generative adversarial network (GAN). The device 100 for converting a medical image may include:
an image quality degrader 300 which degrades the image quality of a second original image selected from a paired data set including a first original image and the second original image to construct a degraded image; and
a learning model 200 which receives the first original image, constructs an output image based on the first original image, the output image matching the first original image and belonging to a different domain from the first original image,
receives the degraded image from the image quality degrader 300, and is trained based on the degraded image to improve the accuracy of a result of medical image conversion.

In some embodiments, the image quality degrader 300 may construct the degraded image by blurring the second original image. In some embodiments, the blurring may include removing high frequency components from the second original image.

In some embodiments the learning model 200 may include a first discriminator 221 which is trained to discriminate the output image from the second original image and a second discriminator 222 which is trained to discriminate the degraded image from the second original image. In some embodiments, the first discriminator 221 may be trained to determine that the output image is different from the second original image, and the second discriminator 222 may be trained, like the first discriminator 221, to determine that the degraded image is different from the second original image.

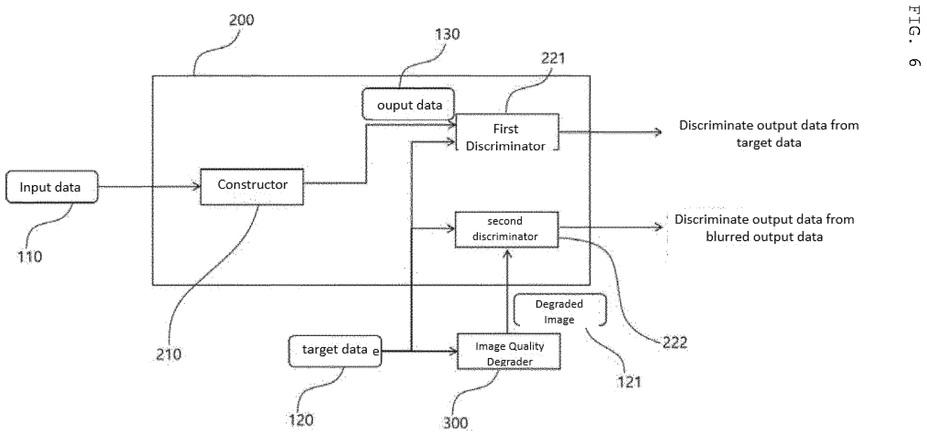

FIG. 6 illustrates an Al learning model according to an embodiment of the disclosure.

An Al learning model 200 according to an embodiment of the disclosure includes a constructor 210 and a discriminator 220, and is trained by machine learning. The Al learning model 200 may include:
a constructor 210 which receives a first original image and is trained to construct an output image based on the first original image using a generative adversarial network (GAN);
a first discriminator 221 which is trained to discriminate the output image constructed by the constructor 210 from a second original image paired with the first original image; and
a second discriminator 222 which is trained to discriminate a degraded image 121 degraded from the second original image from the second original image.

As used in the disclosure, the degraded image 121 may also be a low quality image degraded from a high quality image. For example, the degraded image 121 may include a case in which the clarity, sharpness, resolution, or the like of an image is degraded.

In some embodiments, the degraded image 121 may be constructed by blurring the second original image.

In some embodiments, the blurring may include removing high frequency components from the second original image.

In the training method according to an embodiment of the disclosure, (1) a first constructor constructs an output image from an input image, (2) a discriminator determines that the constructed output image is false, (3) a second constructor inputs an additionally constructed blurred target image into the discriminator, (4) if the discriminator determines the blurred target image to be false, (5) a loss function compares the constructed output image with an original target image to calculate a loss value, (6) the calculated loss value is input again (or fed back) to the constructor, and (7) as the loss value is input again repeatedly, the learning model 200 may be updated. As a result, the discriminator is trained to recognize the output image constructed in the second operation as true.

A model training method for medical image conversion according to another aspect of the disclosure may include a model training method for medical image conversion which converts a medical image using a generative adversarial network (GAN).

The model training method for medical image conversion according to this embodiment the disclosure includes: a first operation of receiving a first original image selected from a paired data set including the first original image and a second original image;
a second operation of constructing an output image based on the first original image, the output image matching the first original image and belonging to a different domain from the first original image;
a third operation of improving the quality of the second original image to construct an improved image; and
a fourth operation of performing training based on the improved image to improve the accuracy of a result of medical image conversion.

Here, by improving the quality of the second original image and providing the improved image as the target image so that the discriminator 220 determines based on the improved image, a clearer output image may be obtained, compared to an output image conventionally produced by comparing the output image with the target image without improved image quality. That is, because the training is performed to improve the quality of the ground-truth target image and then reduce the difference by comparing the output image with the target image with improved quality, a clearer output image may be obtained compared to the conventional training method in which the training is performed to reduce the difference by comparing an output image with a target image without improved quality.

For example, in a case in which a medical image is captured using an MR imaging device, the clarity may vary depending on whether the scan is performed at 3 Tesla or 7 Tesla. Because detailed portions in reconstructed MR images are different depending on the degree of signal sampling, the clarity may be expressed differently.

In a case in which imaging is performed using a weak magnetic field and low signal sampling, the image may be further clarified or sharpened by removing low frequency components.

In the related art, a third image is merely synthesized from a first image, but in the disclosure, the learning model may be trained with a slightly over-sharpened image to produce a second image, i.e., an output image, having a desired level of high resolution.

Because the training result of a GAN model tends to output blurred images, the disclosure allows the training to be performed based on an over-sharpened target image. Even in a case in which a slightly blurred image is constructed from a clear image, an image having a desired level of clarity may be generated, and thus the training may be performed based on an over-sharpened target image.

According to the disclosure, removing low frequency components from the second image forms an overly clear image.

Because the third medical image is the original image, a target image may be constructed by processing the third medical image to increase the resolution, i.e., to remove low frequencies, and then based on the target image, the second image may be processed to produce a result having high clarity.

In this embodiment, removing low frequency components increases the clarity of the removed image itself. In the embodiment described above, removing high frequency components reduces the clarity of the original image, but, when the model is trained by inputting the original image with reduced clarity, the clarity of the converted output image increases instead.

The above-described embodiments of the disclosure may be implemented in the form of computer programs executable on a computer using various components, and such computer programs may be stored in computer-readable media. Examples of the computer-readable media may include: magnetic media such as hard disks, floppy disks, and magnetic tapes; optical recording media such as CD-ROMs and DVDs; magneto-optical media such as floptical disks; and hardware devices such as ROMs, RAMs, and flash memories specifically configured to store program instructions and execute the program instructions.

Furthermore, the computer programs may be specifically designed and configured for the disclosure or may be known and available to a person having ordinary knowledge in the art of computer software. Examples of computer programs may include machine code produced by compilers and high-level language code executable on computers using interpreters.

In the specification of the disclosure, the use of the term "the" and similar denoting terms may correspond to both singular and plural forms. Furthermore, recitation of ranges of values herein are intended merely to refer to respective separate values falling within the respective ranges and, unless otherwise indicated herein, the respective separate values are incorporated herein as if individually recited herein.

Finally, the operations of any method described herein may be performed in any suitable order unless otherwise indicated herein or otherwise clearly contradicted by the context. However, the operations shall not be limited to the described sequence. The use of any examples or illustrative languages (e.g., "such as") provided herein, is intended merely to better illustrate the disclosure and does not pose a limitation on the scope of the disclosure unless otherwise defined by the Claims. Furthermore, a person having ordinary knowledge in the art will appreciate that various modifications, combinations, and changes are possible according to design conditions and factors within the scope of the Claims or equivalents thereof.

Therefore, the spirit of the disclosure shall not be limited to the above-described embodiments, and the entire scope of the appended claims and equivalents thereof will fall within the scope and spirit of the disclosure.

### <Description of Reference Numerals of Drawings>

100: device for converting a medical image
110: first image
120: second image
121: degraded image
130: third image
200: Al learning model
210: constructor
220: discriminator
221: first discriminator
222: second discriminator
300: image quality degrader

## Claims

1. A model training method for medical image conversion which converts a medical image using a generative adversarial network (GAN), the model training method comprising:
a first operation of receiving a first original image selected from a paired data set including the first original image and a second original image;
a second operation of constructing an output image based on the first original image, the output image matching the first original image and belonging to a different domain from the first original image;
a third operation of degrading quality of the second original image to construct a degraded image; and
a fourth operation of performing training based on the degraded image to improve accuracy of a result of medical image conversion.

2. The model training method of claim 1, wherein in the third operation, the degraded image is constructed by blurring the second original image.

3. The model training method of claim 2, wherein the blurring comprises removing high frequency components from the second original image.

4. The model training method of claim 1, wherein the fourth operation comprises:
a first discrimination operation of discriminating the output image from the second original image; and
a second discrimination operation of discriminating the degraded image from the second original image.

5. The model training method of claim 4, wherein in the first discrimination operation, the training is performed to determine that the output image is different from the second original image, and in the second discrimination operation, the training is performed to determine that the degraded image is different from the second original image.

6. A device for converting a medical image using a generative adversarial network (GAN), the device comprising:
an image quality degrader configured to degrade image quality of a second original image selected from a paired data set comprising a first original image and the second original image to construct a degraded image; and
a learning model configured to receive the first original image, construct an output image based on the first original image, the output image matching the first original image and belonging to a different domain from the first original image, receive the degraded image from the image quality degrader, and be trained based on the degraded image to improve accuracy of a result of medical image conversion.

7. The device of claim 6, wherein the image quality degrader constructs the degraded image by blurring the second original image.

8. The device of claim 7, wherein the blurring comprises removing high frequency components from the second original image.

9. The device of claim 6, wherein the learning model comprises a first discriminator configured to be trained to discriminate the output image from the second original image and a second discriminator configured to be trained to discriminate the degraded image from the second original image.

10. The device of claim 9, wherein the first discriminator is trained to determine that the output image is different from the second original image, and the second discriminator is trained, like the first discriminator, to determine that the degraded image is different from the second original image.

11. An artificial intelligence learning model which comprises a constructor and a discriminator and is trained by machine learning, the Al learning model comprising:
a constructor configured to receive a first original image and be trained to construct an output image based on the first original image using a generative adversarial network (GAN);
a first discriminator configured to be trained to discriminate the output image constructed by the constructor from a second original image paired with the first original image; and
a second discriminator configured to be trained to discriminate the degraded image degraded from the second original image from the second original image.

12. The artificial intelligence learning model of claim 11, wherein the degraded image is constructed by blurring the second original image.

13. The artificial intelligence learning model of claim 12, wherein the blurring comprises removing high frequency components from the second original image.

14. The artificial intelligence learning model of claim 11, wherein the first discriminator is trained to determine that the output image is different from the second original image, and the second discriminator is trained, like the first discriminator, to determine that the degraded image is different from the second original image.

15. A model training method for medical image conversion which converts a medical image using a generative adversarial network (GAN), the model training method comprising:
a first operation of receiving a first original image selected from a paired data set including the first original image and a second original image;
a second operation of constructing an output image based on the first original image, the output image matching the first original image and belonging to a different domain from the first original image;
a third operation of improving quality of the second original image to construct an improved image; and
a fourth operation of performing training based on the improved image to improve accuracy of a result of medical image conversion.
